# EUROPÄISCHE PATENTSCHRIFT

(11) **EP 2 745 832 B1**
(45) Veröffentlichungstag und Bekanntmachung des Hinweises auf die Patenterteilung: **09.11.2016**
(21) Anmeldenummer: 13196362.1
(22) Anmeldetag: 10.12.2013
(51) Int. Cl.: A61K 8/44, A61K 8/73, A61Q 19/08, A61K 8/97

(54) **Hautpflegemittel zur Behandlung reifer Haut**
Skin care composition for treating mature skin
Produit de soin cutané pour le traitement des peaux matures

(30) Priorität: 18.12.2012 DE 102012223491
(43) Veröffentlichungstag der Anmeldung: 25.06.2014
(73) Patentinhaber: Henkel AG & Co. KGaA, 40589 Düsseldorf (DE)
(72) Erfinder: Keßler-Becker, Daniela, 51371 Leverkusen (DE); Dickhof, Susanne, 41748 Viersen (DE)

(56) Entgegenhaltungen:
- DE-A1-102008 006 844
- DE-A1-102008 053 273
- DE-A1-102008 061 340
- DE-C1- 3 942 726
- Anonymous: "GNPD - Intense Moisturising Mask", , 1. November 2012 (2012-11-01), XP055118519, Gefunden im Internet: URL:http://www.gnpd.com/sinatra/recordpage /1908829/from_search/ZvnR1F2h7g/ [gefunden am 2014-05-16]
- Anonymous: "GNPD - Moisturizing Cream (Rich)", , 1. November 2011 (2011-11-01), XP055118522, Gefunden im Internet: URL:http://www.gnpd.com/sinatra/recordpage /1735016/from_search/ZvnR1F2h7g/ [gefunden am 2014-05-16]
- Anonymous: "GNPD - Moisturizing Toner", , 1. November 2011 (2011-11-01), XP055118521, Gefunden im Internet: URL:http://www.gnpd.com/sinatra/recordpage /1734534/from_search/ZvnR1F2h7g/ [gefunden am 2014-05-16]

## Beschreibung

Gegenstand der vorliegenden Anmeldung sind kosmetische Zusammensetzungen zur topischen Behandlung der Haut, die einen Ester der Hyaluronsäure, ein spezielles Betain und einen Extrakt aus den Samen des Johannisbrotbaums enthalten.

Gegenstand der vorliegenden Erfindung ist weiterhin die nicht-therapeutische Verwendung der zuvor genannten kosmetischen Zusammensetzung.

Im Laufe des Alterns verändert sich die Beschaffenheit der obersten Hautschicht, der Epidermis. Sie wird dünner und die Verbindungen zwischen den Zellen lockern auf. Dadurch ist die Altershaut gegenüber äußeren Einflüssen, wie mechanischem Stress oder Klimaeffekten, besonders anfällig. Die Haut ist leicht verwundbar, reagiert sensibel gegenüber äußeren Reizen und trocknet schneller aus. Verbraucher suchen nach Gesichtspflegeprodukten, die neben einem sofort erlebbaren Effekt auch die Beschaffenheit der Haut über einen längeren Zeitraum sichtbar und fühlbar verbessern. Insbesondere Verbraucher mit reiferer Haut klagen häufig über trockene Haut, die des Weiteren leicht durch mechanische Einflüsse verletzt werden kann und durch Reizungen häufiger juckt oder brennt.

In der Vergangenheit wurde eine Vielzahl kosmetischer Produkte speziell für die Behandlung reifer Haut vorgeschlagen. Die überwiegende Zahl dieser Produkte enthält bekannte und bewährte Feuchthaltemittel und/oder natürliche Feuchthaltefaktoren (natural moisturizing factors), die reife Haut vor der Austrocknung bewahren und/oder die Hautbarriere stärken.

Unter die zahlreichen bekannten Feuchthaltemittel fallen u.a. Hyaluronsäure und/oder deren Derivate und/oder Salze sowie niedrigmolekulare Betaine (beispielsweise WO 2010/049389 und US 2006/0030621).

In der Offenlegungsschrift WO 2011/077017 werden bestimmte Peptidextrakte, erhältlich aus Keimen des Johannisbrotbaums (Ceratonia Siliqua), für die Behandlung besonders trockener Haut und zur Stärkung der Hautbarriere vorgeschlagen.

Dokument DE 102008053273A1 betrifft topische Zusammensetzungen zur Behandlung von Hautfalten, die Hyaluronsäure und einen Apfelkernextrakt als Wirkstoffkombination enthalten.

Die bekannten Produkte können jedoch nicht alle Bedürfnisse der Verbraucher ausreichend abdecken. Sie eignen sich zwar überwiegend für die Behandlung der Symptome reifer Haut (Verhinderung des Wasserverlustes und/oder der Milderung von Hautfalten), doch wünscht sich der Verbraucher effektivere Produkte, die nicht nur kurzfristig die Hautfeuchtigkeit erhöhen und/oder die sichtbaren Zeichen der Hautalterung mildern. Vielmehr besteht der Bedarf nach Produkten,

die die Befeuchtung, die Straffung, die Barriereeigenschaften und das optische Gesamtbild der Haut "von innen heraus" unterstützen. Darunter wird verstanden, dass die Produkte optimalerweise die natürlichen Erneuerungs- und Reparierungsprozesse in der Haut aktivieren, und damit den Feuchtigkeitsgehalt der Haut nachhaltig verbessern bzw. den Alterungsprozess der Haut verzögern.

Der vorliegenden Anmeldung lag die Aufgabe zu Grunde, ein kosmetisches Hautpflegemittel bereitzustellen, das den Feuchtigkeitsgehalt und die Barrierefunktion der Haut nachhaltig verbessert Weitere Ziele sind die Verbesserung der Hautfestigkeit und die Milderung von Linien, Falten und/oder Fältchen der Haut. Insbesondere sollen sich die Hautpflegemittel für die Behandlung reiferer Haut (d.h. von Haut - insbesondere menschlicher Gesichtshaut - eines Alters > 40 Jahre) eignen.

Überraschenderweise führte die Kombination von Hyaluronsäure (bzw. einem Derivat oder einem Salz der Hyaluronsäure), einem bestimmten Betain und einem Extrakt aus den Samen des Johannisbrotbaums in topischen Hautbehandlungsmitteln zu einer signifikanten Erhöhung der Hautfeuchtigkeit sowie zu einer Verbesserung der Hautstruktur.

Ein erster Gegenstand der vorliegenden Anmeldung ist daher eine kosmetische Zusammensetzung zur topischen Behandlung der Haut, die in einem geeigneten kosmetischen Träger (jeweils bezogen auf das Gesamtgewicht der Zusammensetzung)
a) 0,01 bis 20 Gew.-% mindestens eines Esters der Hyaluronsäure,
b) 0,01 bis 20 Gew.-% mindestens einer Verbindung der nachfolgenden Formel (I) 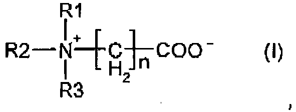 die ein Molekulargewicht von 75 bis 200 aufweist, und
   in der
   n für eine Zahl von 1 bis 3 und
   die Reste R1, R2 und R3 unabhängig voneinander für ein Wasserstoffatom, eine Methylgruppe, eine Ethylgruppe oder eine C₂-C₄-Hydroxyalkylgruppe stehen, und
c) 0,01 bis 20 Gew.-% mindestens eines wässrigen oder wässrig-alkoholischen Extrakts aus den Samen des Johannisbrotbaums *(Ceratonia siliqua)* enthält.

Die erfindungsgemäßen Zusammensetzungen weisen gegenüber bekannten Hautpflegemitteln den Vorteil auf, dass sie die sichtbaren Zeichen der Hautalterung nachhaltig mildern, indem sie biologische Mechanismen in der Epidermis stimulieren.

Die Wirkstoffkombination in den erfindungsgemäßen Zusammensetzungen führte u.a. jeweils zu einer erhöhten Expression
- des Proteins Filaggrin (welches für die Stärkung der natürlichen Hautbarriere verantwortlich ist),
- des Intermembran-Proteins Aquaporin-3 (welches für die Aufrechterhaltung des Wasserhaushalts in der Epidermis verantwortlich ist), und
- des Tight-Junction-Proteins Claudin-1 (welches für die Stärkung der Zell-Zell-Verbindungen in der Epidermis und damit für eine erhöhte mechanische Festigkeit der Haut verantwortlich ist). Ein verbesserter Zellverband schützt besonders reifere Haut vor mechanischen Verletzungen. Des Weiteren können exogene Noxen, wie Chemikalien oder Mikroben, schwerer durch einen gefestigten Zellverband in die Haut eindringen und zu negativen Effekten führen.

Darüber hinaus konnte mithilfe der erfindungsgemäßen Wirkstoffkombination die hauteigene Hyaluronsäureproduktion gesteigert werden, wodurch der altersbedingte Volumenabbau der Haut verringert werden konnte.

Die erfindungsgemäßen Zusammensetzungen enthalten die Wirkstoffkombination a), b), c) in einem geeigneten kosmetischen Träger. Darunter wird bevorzugt ein topischer Träger verstanden, der bevorzugt ein wässriger oder wässrig-alkoholischer Träger ist. Bevorzugt enthält der Träger - bezogen auf sein Gesamtgewicht - mindestens 20 Gew.-%, mehr bevorzugt mindestens 25 Gew.-% besonders bevorzugt mindestens 30 Gew.-% und insbesondere mindestens 35 Gew.-% Wasser. Weiterhin kann der kosmetische Träger 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, 1-Propanol, 2-Propanol, Isopropanol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole. Bevorzugt sind die wasserlöslichen Alkohole. Besonders bevorzugt sind Ethanol, 1-Propanol, 2-Propanol, 1,2-Propylenglycol, Glycerin, und/oder 1,6-Hexandiol sowie Mischungen dieser Alkohole. Insbesondere bevorzugt sind Glycerin und/oder 1,6-Hexandiol.

Ein besonders bevorzugter Träger enthält mindestens 35 Gew.-% Wasser und mindestens 5 Gew.-% Glycerin und/oder 1,6-Hexandiol.

Erfindungsgemäß bevorzugte Zusammensetzungen sind dadurch gekennzeichnet, dass sie den mindestens einen Wirkstoff a), ausgewählt aus Estern der Hyaluronsäure, in einer Gesamtmenge von 0,05 bis 15 Gew.-%, weiter bevorzugt von 0,1 bis 10 Gew.-%, besonders bevorzugt von 0.5 bis 7,5 Gew.-% und insbesondere von 1 bis 5 Gew.-%, jeweils bezogen auf das Gesamtgewicht der Zusammensetzung, enthalten.

Bevorzugte Ester der Hyaluronsäure sind ausgewählt aus Sllanolestern der Hyaluronsaure, wie sie beispielsweise unter der INCI-Bezeichnung Dimethylsilanol Hyaluronate erhältlich sind. Ein bevorzugtes Handelsprodukt wird beispielsweise von der Firma Exsymol unter der Bezeichnung D.S.H. CN^{®} oder D.S.H. CN^{®} im Handel angeboten.

In einer bevorzugten Ausführungsform enthalten erfindungsgemäße Zusammensetzungen als Wirkstoff a) daher mindestens einen Silanolester der Hyaluronsäure, besonders bevorzugt Dimethylsilanediol hyaluronate (INCI-Bezeichnung: Dimethylsilanol Hyaluronate).

Ein weiterer wesentlicher Inhaltsstoff der erfindungsgemäßen Zusammensetzungen ist mindestens eine Verbindung der Formel (I). 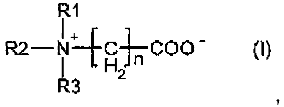 die ein Molekulargewicht von 75 bis 200 aufweist, und in der
n für eine Zahl von 1 bis 3 steht, und
die Reste R1, R2 und R3 unabhängig voneinander für ein Wasserstoffatom, eine Methylgruppe, eine Ethylgruppe oder eine C₂-C₄-Hydroxyalkylgruppe stehen.

Bevorzugte Verbindungen der Formel (I) weisen ein Molekulargewicht von 90 bis 200 und Insbesondere von 100 bis 150 auf.

In weiterhin bevorzugten Verbindungen gemäß Formel (I) stehen die Reste R1, R2 und R3 jeweils für eine Methylgruppe und n steht für die Zahl 1.

Insbesondere bevorzugt ist demnach Trimethylglycin, das im Handel unter der INCI-Bezeichnung Betain von mehrenen Anbietern erhältlich ist (z.B. Betafin® BP 20 - Finnfeeds; Betain^{®} NMF 50 - Beijing Onlystar; Tego Natural Betaine^{®} - Evonik).

Die erfindungsgemäßen Zusammensetzungen enthalten Verbindung(en) der zuvor genannten Formel (I) bevorzugt in einer Gesamtmenge von 0,05 bis 15 Gew.-%, weiter bevorzugt von 0,1 bis 10 Gew.-%, besonders bevorzugt von 0,5 bis 7,5 Gew.-% und insbesondere von 1 bis 5 Gew.-%, jeweils bezogen auf das Gesamtgewicht der Zusammensetzung.

Als dritten wesentlichen Bestandteil enthalten die erfindungsgemäßen Zusammensetzungen mindestens einen wässrigen oder wässrig-alkoholischen Extrakt aus den Samen des Johannisbrotbaums (*Ceratonia Siliqua*)*.*

Der Johannisbrotbaum (Karubembaum, Carobbaum) ist eine Pflanzenart aus der Familie der Hülsenfrüchtler. Er ist überwiegend im Mittelmeerraum sowie in Vorderasien beheimatet.

Die Keime aus den Samen des Johannisbrotbaums sind besonders proteinreich und werden daher besonders bevorzugt als Extraktionsgrundlage für die erfindungsgemäßen Zusammensetzungen genutzt.

Prinzipiell können erfindungsgemäß geeignete Extrakte aus den Samen des Johannisbrotbaums nach allen dem Fachmann bekannten Extraktionsverfahren gewonnen werden.

Erfindungsgemäß bevorzugt sind jedoch Extrakte, die nach einem Verfahren erhältlich sind, welches in der Offenlegungsschrift WO 2011/07717 beschrieben wird.

Die wässrigen oder wässrig-alkoholischen Extrakte aus den Samen des Johannisbrotbaums *(Ceratonia siliqua)* werden bevorzugt in einer Gesamtmenge von 0,05 - 15 Gew.-%, weiter bevorzugt von 0,1 -10 Gew.-%, besonders bevorzugt von 0.25 - 7,5 Gew.-% und insbesondere von 0,5 - 5 Gew.-%, jeweils bezogen auf das Gesamtgewicht der Zusammensetzung, in den erfindungsgemäßen Zusammensetzungen eingesetzt.

Insbesondere bevorzugt sind wässrig-alkoholische Extrakte aus den Samen des Johannisbrotbaums *(Ceratonia siliqua),* die einen Proteingehalt im Bereich von 0,5 bis 10 g/l, bevorzugt von 1 bis 5 g/l und insbesondere von 1,5 bis 3,5 g/l in einer Mischung aus Wasser und einem Polyol, bevorzugt Glycerin, aufweisen.

Besonders geeignete wässrige oder wässrig-alkoholische Samenextrakte des Johannisbrotbaums sind im Handel erhältlich, beispielsweise von der Firma ISP (Vincience) unter der Bezeichnung "Aqua-Osmoline^{®} (INCI-Bezeichnung: Water, Glycerin, Ceratonia Siliqua Seed Extract).

In einer ersten besonders bevorzugten Ausführungsform enthalten erfindungsgemäße Zusammensetzungen - bezogen auf ihr Gesamtgewicht-
a) 0,05 bis 15 Gew.-% mindestens eines Esters der Hyaluronsäure,
b) 0,05 bis 15 Gew.-% mindestens einer Verbindung der zuvor genannten Formel (I), die ein Molekulargewicht von 75 bis 250, bevorzugt von 90 bis 200 und insbesondere von 100 bis 150 aufweist, und
c) 0,05 bis 15 Gew.-% mindestens eines wässrigen oder wässrig-alkoholischen Extrakts aus den Samen des Johannisbrotbaums:

Innerhalb dieser Ausführungsform sind solche erfindungsgemäßen Mittel insbesondere bevorzugt, die - bezogen auf ihr Gesamtgewicht -
a) 0,1 bis 10 Gew.-% mindestens eines unter der INCI-Bezeichnung Dimethylsilanol Hyaluronate bekannten Hyaluronsäureesters,
b) 0,1 bis 10 Gew.-% Trimethylglycin und
c) 0,1 bis 10 Gew.-% mindestens eines wässrig-alkoholischen Extrakts aus den Samen des Johannisbrotbaums enthalten, welche einen Proteingehalt von 0,5 bis 10 g/l, bevorzugt von 1 bis 5 g/l und insbesondere von 1,5 bis 3,5 g/l in einer Mischung aus Wasser und einem Polyol, bevorzugt Glycerin, aufweisen.

Erfindungsgemäße kosmetische Zusammensetzungen können als Pasten, Salben, Lotionen oder Cremes vorliegen. Feste Mittel können beispielsweise als Stift vorliegen.

Vorteilhafterweise liegen die erfindungsgemäßen Zusammensetzungen zur topischen Behandlung der Haut in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, oder einer Mischung mit mindestens einem als medizinischen Klebstoff geeigneten Polymer vor.

Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

Besonders bevorzugte erfindungsgemäße Zusammensetzungen liegen in Form einer fließfähigen Emulsion oder eines wässrigen Gels, insbesondere in Form einer Öl-in-Wasser-Emulsion, einer Wasser-in-Öl und/oder eines wässrigen Gels vor.

Erfindungsgemäß besonders geeignete Emulsionen enthalten neben den bereits beschriebenen Wirkstoffen bevorzugt mindestens einen weiteren Hautkonditionierenden Wirkstoff und mindestens einen Emulgator.

Unter geeigneten Hautkonditionierenden Wirkstoffen sind bevorzugt solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Bevorzugte Hautkonditionierende Wirkstoffe können ausgewählt sein aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsaeuren, Fettalkoholen, besonders gesättigten, ein- oder mehrfach ungesättigten, verzweigten oder unverzweigten Fettalkoholen mit 4-30 Kohlenstoffatomen, die mit 1-75, bevorzugt 5-20 Ethylenoxid-Einheiten ethoxyliert und/oder mit 3-30, bevorzugt 9-14 Propylenoxid-Einheiten propoxyliert sein können, Esterölen, das heißt Estern von C₆₋₃₀-Fettsaeuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1-10, bevorzugt 7- 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesaettigter und gegebenenfalls hydroxylierter C₁₆₋₃₀-Fettsaeuren, z. B. gehärteten Triglyceridfetten, Phospholipiden, beispielsweise Sojalecithin, Ei-Lecithin und Kephalinen, Shea-Butter, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin-und/oder Hydroxy-Gruppen enthalten, bevorzugt Substanzen mit den INCI-Bezeichnungen Dimethiconol, Amodimethicone oder Trimethylsilylamodimethicone. In einer bevorzugten Ausführungsform enthalten die erfindungsgemäßen Zusammensetzungen eine Mischung von Hautkonditionierenden Wirkstoffen aus der zuvor genannten Liste.

Die Einsatzmenge der Hautkonditionierenden Wirkstoffe in den erfindungsgemäßen Zusammensetzungen beträgt bevorzugt 0,1-80 Gew.-%, besonders bevorzugt 1-50 Gew.-% und außerordentlich bevorzugt 5-20 Gew.-%, jeweils bezogen auf die gesamte Zusammensetzung.

Geeignete oberflächenaktive Substanzen und/oder Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsaeuren und an C₈-C₁₅-Alkylphenole, C₁₂-C₂₂-Fettsäuremono- und - diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, z.B. die unter das unter der INCI-Bezeichnung Coco Glucoside bekannte Produkt, Gemische aus Alkyl-(oligo)-glucosiden und Fettalkoholen, z. B. die im Handel erhältlichen Produkte Montanov^{®}68 und Montanov^{®} 202, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₁₂-Fettsaeuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsaeureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsaeuren und deren Na-, K-, Ammonium-, Ca-, Mg-und Zn-Salze.

Die erfindungsgemäßen Zusammensetzungen können die Emulgatoren bevorzugt in Mengen von 0,1 bis 25 Gew.-%, besonders bevorzugt von 0,5-15 Gew.-% und insbesondere von 1 bis 10 Gew.-%, bezogen auf die gesamte Zusammensetzung, enthalten.

Weitere erfindungsgemäß besonders bevorzugte kosmetische Zusammensetzungen sind dadurch gekennzeichnet, dass sie weiterhin mindestens einen Hautberuhigenden Wirkstoff enthalten. Erfindungsgemäß bevorzugte Hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.

Die Hautberuhigenden Wirkstoffe sind bevorzugt in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Weitere geeignete Zusatzstoffe erfindungsgemäß bevorzugter Emulsionen und/oder wässriges Gele sind Verdickungsmittel, z. B. anionische Polymere aus Acrylsäure, Methacrylsäure, Crotonsäure, Maleinsäureanhydrid und 2-Acrylamido-2-methylpropansulfonsaeure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS und Simulgel^{®} EPG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80-98% eine ungesättigte, gewünschtenfalls substituierte C₃₋₆-Carbonsäure oder ihr Anhydrid sowie zu 2-20% gewünschtenfalls substituierte Acrylsäureester von gesättigten C₁₀₋₃₀-Carbonsaeuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen. Vitamine, Pro-Vitamine und Vitaminvorstufen sind dabei besonders bevorzugt, die den Gruppen A, B, C, E, F und H zugeordnet werden. Zur Gruppe der als Vitamin A bezeichneten Substanzen gehören das Retinol sowie das 3,4-Didehydroretinol. Das beta-Carotin ist das Provitamin des Retinols. Als Vitamin-A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung. Zur Vitamin B-Gruppe oder zu dem Vitamin B-Komplex gehören u. a.: Vitamin B-1 (Thiamin) Vitamin B-2 (Riboflavin) Vitamin B-3: Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist. Vitamin B-5: (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Pantothensäure wird bevorzugt als Derivat in Form der stabileren Calciumsalze und Natriumsalze (Ca-Pantothenat, Na-Pantothenat) in der vorliegenden Erfindung eingesetzt. Vitamin B-6 (Pyridoxin sowie Pyridoxamin und Pyridoxal). Die genannten Verbindungen des Vitamin B -Typs insbesondere Vitamin B-3, B-5 und B-6, sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt. Vitamin C (Ascorbinsäure). Vitamin C wird in den erfindungsgemäßen Mitteln bevorzugt in Mengen von 0, 1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein. Vitamin E (Tocopherole, insbesondere .alpha.-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten. Vitamin F. Unter dem Begriff "Vitamin F" werden üblicherweise essentielle Fettsäuren, insbesondere Linolsäure, Linolensäure und Arachidonsäure, verstanden. Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und/oder H. Panthenol, Pantolacton, Pyridoxin und seine Derivate sowie Nicotinsäureamid und Biotin sind besonders bevorzugt.

In einer zweiten besonders bevorzugten Ausführungsform liegen die erfindungsgemäßen Zusammensetzungen als Öl-in-Wasser-Emulsion oder Wasser-in-Öl-Emulsion vor und enthaltenbezogen auf ihr Gesamtgewicht -
a) 0,01 bis 20 Gew.-%, bevorzugt 0,05 bis 15 Gew.-% mindestens eines Esters der Hyaluronsäure,
b) 0,01 bis 20 Gew.-%, bevorzugt 0,05 bis 15 Gew.-% mindestens einer Verbindung der zuvor genannten Formel (I), die ein Molekulargewicht von 75 bis 250, bevorzugt von 90 bis 200 und insbesondere von 100 bis 150 aufweist,
c) 0,01 bis 20 Gew.-%, bevorzugt 0,05 bis 15 Gew.-% mindestens eines wässrigen oder wässrig-alkoholischen Extrakts aus den Samen des Johannisbrotbaums,
d) 1 bis 50 Gew.-%, bevorzugt 5 bis 20 Gew.-% mindestens eines Hautkonditionierenden Wirkstoffs,
e) 0,1 bis 25 Gew.-%, bevorzugt 0,5-15 Gew.-% mindestens eines Emulgators und
f) 0,01 bis 10 Gew.-%, bevorzugt 0,05 bis 5 Gew.-% mindestens eines Hautberuhigenden Wirkstoffs und/oder mindestens eines Vitamins, Provitamins und/oder einer Vitaminvorstufe.

Innerhalb dieser Ausführungsform sind solche erfindungsgemäßen O/W- oder W/O-Emulsionen insbesondere bevorzugt, die - bezogen auf ihr Gesamtgewicht -
a) 0,1 bis 10 Gew.-% mindestens eines unter der INCI-Bezeichnung Dimethylsilanol Hyaluronate bekannten Hyaluronsäureesters,
b) 0,1 bis 10 Gew.-% Trimethylglycin,
c) 0,1 bis 10 Gew.-% mindestens eines wässrig-alkoholischen Extrakts aus den Samen des Johannisbrotbaums, welche einen Proteingehalt von 0,5 bis 10 g/l, bevorzugt von 1 bis 5 g/l und insbesondere von 1,5 bis 3,5 g/l in einer Mischung aus Wasser und einem Polyol, bevorzugt Glycerin, aufweisen,
d) 5-20 Gew.-% mindestens einen Hautkonditionierenden Wirkstoffs, der Shea-Butter, Silikone, Fettsäure(di)carbonate und/oder Fettsäuretriglyceride enthält,
e) 1 bis 10 Gew.-% mindestens eines Emulgators, bevorzugt eines Alkyl-(oligo)-glucosids,
f) 0,05 bis 5 Gew.-% mindestens eines Hautberuhigenden Wirkstoffs, ausgewählt aus alpha-Bisabolol und/oder Allantoin, und
g) 0,05 bis 5 Gew.-% mindestens eines Vitamins, Provitamins und/oder einer Vitaminvorstufe aus den aus den Gruppen A, B, E und H, bevorzugt Panthenol, Pantolacton, Pyridoxin, Nicotinsäureamid und/oder Biotin enthalten.

Weitere geeignete Zusatzstoffe erfindungsgemäß bevorzugter O/W-, W/O-Emulsionen und/oder wässriger Gele sind beispielsweise:
- Parfumöle,
- Substanzen zur Einstellung des pH-Wertes,
- Komplexbildner wie EDTA, NTA, β-Alanindiessigsaeure und Phosphonsaeuren,
- Treibmittel wie Propan-Butan-Gemische, Pentan, Isopentan, Isobutan, N₂O, Dimethylether, CO₂ und Luft,
- Wirkstoffe wie Glycyrrhizinsäure bzw. deren physiologisch verträglich Salze und/oder Derivate,
- Pigmente,
- Konservierungsmittel wie Phenoxyethanol und/oder Parabene und/oder
- Abrasivstoffe.

Wie bereits ausgeführt, eignen sich die erfindungsgemäßen Zusammensetzungen besonders für die Behandlung reiferer Haut, da sie Alterungserscheinungen der Haut nachhaltig bekämpft.

Ein zweiter Gegenstand der Erfindung ist daher nicht-therapeutische, kosmetische Verwendung einer kosmetischen topischen Zusammensetzung gemäß des ersten Erfindungsgegenstandes, die in einem geeigneten kosmetischen Träger (jeweils bezogen auf das Gesamtgewicht der Zusammensetzung)
a) 0,01 bis 20 Gew.-% mindestens eines Esters der Hyaluronsäure,
b) 0,01 bis 20 Gew.-% mindestens einer Verbindung der nachfolgenden Formel (I) 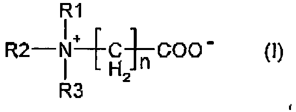 die ein Molekulargewicht von 75 bis 200 aufweist, und in der
   n für eine Zahl von 1 bis 3 und
   die Reste R1, R2 und R3 unabhängig voneinander für ein Wasserstoffatom, eine Methylgruppe, eine Ethylgruppe oder eine C₂-C₄-Hydroxyalkylgruppe stehen, und
c) 0,01 bis 20 Gew.-% mindestens eines wässrigen oder wässrig-alkoholischen Extrakts aus den Samen des Johannisbrotbaums *(Ceratonia siliqua)* enthält,
   - zur Steigerung der mechanischen Festigkeit der Epidermis,
   - zur Verbesserung der Barriereeigenschaften der Epidermis,
   - zur nachhaltigen Steigerung der Hautfeuchte,
   - zur Verbesserung der Zell-Zell-Verbindungen in der Epidermis,
   - zum Schutz der Epidermis vor mechanischen Verletzungen und/oder vor exogenen Noxen,
   - zur nicht-therapeutischen, kosmetischen Behandlung intrinsisch und/oder extrinsisch gealterter Haut, insbesondere zur Antifaltenbehandlung, und
   - zur Glättung der Haut und/oder zur Verminderung von Falten, Fältchen und/oder feinen Linien und/oder zur Verbesserung des optischen Erscheinungsbildes der Haut.

Die Steigerung der Hautfeuchte, der mechanischen Festigkeit der Epidermis, der Verbesserung der Barriereeigenschaften und der Zell-Zell-Verbindungen in der Epidermis sowie die Hautglättung und Milderung von Fältchen konnte erreicht werden, indem die hauteigene Hyaluronsäureproduktion sowie die Produktion der Hautproteine Filaggrin, Aquaporin-3 und Claudin-1 signifikant gesteigert wurde.

In einer bevorzugten Ausführungsform werden die Zusammensetzungen des ersten Erfindungsgegenstandes demnach bevorzugt zur nicht-therapeutischen, kosmetischen Steigerung der hauteigenen Hyaluronsäureproduktion sowie zur Steigerung der Produktion der Proteine Filaggrin, Aquaporin-3 und Claudin-1 verwendet.

### Experimenteller Teil - Beispiele

### 1) Immunhistologischer Nachweis von epidermalen Markern (Tabelle 1)

Epidermale Hautmodelle wurden topisch mit unterschiedlichen kosmetischen Formulierungen behandelt. 48 Stunden nach Applikation wurden die Modelle isoliert und die Proteinmenge sowie die Proteinverteilung im Hautquerschnitt mittels immunhistologischer Verfahren analysiert.

Auf Proteinebene lassen sich wichtige epidermale Komponenten nachweisen.

Die nachfolgende Tabelle 1 zeigt, dass qualitativ eine erhöhte Expression der Proteine Filaggrin, Aquaporin-3 sowie des Tight-Junction Proteins Claudin-1 im Vergleich zu einer unbehandelten Kontrolle nachgewiesen werden konnte.

**Tabelle 1**

| | Placebo-behandelte Kontrolle (Formel mit Trimethylglycin und Dimethylsilanol Hyaluronate) | Formel mit Trimethylglycin, Dimethylsilanol Hyaluronate und einem wässrig-alkoholischen Samenextrakt des Johannisbrotbaums |
|---|---|---|
| Filaggrin | + | +++ |
| Aquaporin-3 | + | ++ |
| Claudin-1 | + | ++ |

| | | |
|---|---|---|
| + messbare Expression ++ erhöhte Expression +++ stark erhöhte Expression | | |

### 2) Proteinanalyse der Expression von Hyaluronsäure, Filaggrin und Claudin-1 via ex-vivo Biopsien (Tabelle 2)

Ex-vivo Biopsien (Durchmesser 6 mm) wurden topisch mit unterschiedlichen kosmetischen Formulierungen behandelt. 24 und 48 Stunden nach der Applikation wurde die Proteinexpression mittels Immunfluoreszenzfärbung analysiert, und mittels einer optischen Software quantifiziert. Das Ergebnis ist in der nachfolgenden Tabelle dargstellt.

Es wurde gefunden, dass Formel (II) alleine bereits zu einer gesteigerten Produktion von Hyaluronsäure, Claudin-1 und Filaggrin führt.

Auch Formel (I) führt bereits alleine zu einer gesteigerten Hyaluronsäureproduktion.

Die Kombination des Extraktes mit einer Mischung aus Trimethylglycin und Dimethylsilanol Hyaluronate führte jedoch zu einer signifikanten, synergistischen Steigerung der Produktion von Hyaluronsäure, Claudin-1 und Filaggrin (Spalte 3 der Tabelle).

**Tabelle 2**

| | Unbehandelte Kontrolle | I | II | III |
|---|---|---|---|---|
| Hyaluronsäure (48h) | 100% | 132% | 138% | 159% |
| Claudin-1 (48h) | 100% | nicht signifikant | 148% | 158% |
| Filaggrin (24h) | 100% | nicht signifikant | 117% | 138% |

| | | | | |
|---|---|---|---|---|
| I: Formel mit Trimethylglycin, Dimethylsilanol Hyaluronate (Wirkstoffkombination a) und b)), II: Formel mit einem wässrig-alkoholischen Samenextrakt des Johannisbrotbaums (Wirkstoff c)) III: Formel mit Trimethylglycin, Dimethylsilanol Hyaluronate und einem wässrig-alkoholischen Samenextrakt des Johannisbrotbaums (Wirkstoffkombination a), b), c)) | | | | |

### 3) Formulierungsbeispiele

Die nachfolgende Tabelle (Tabelle 3) enthält die Zusammensetzungen der Formeln I, II und III, deren Wirkung unter Punkt 2 beschrieben wurde. Die Mengenangaben beziehen sich jeweils auf Gew.-%.

**Tabelle 3**

| **Rohstoffe** | **I** | **II** | **III** |
|---|---|---|---|
| Montanov^{®} 202 | 5,00 | 5,00 | 5,00 |
| Capryl-/Caprinsäure-Triglycerid | 10,00 | 10,00 | 10,00 |
| Cetiol^{®} CC | 3,00 | 3,00 | 3,00 |
| Cocoglycerides | 2,00 | 2,00 | 2,00 |
| Vitamin-E Acetate | 0,50 | 0,50 | 0,50 |
| Controx^{®} KS C | 0,05 | 0,05 | 0,05 |
| Cetiol^{®} SB 45 | 1,50 | 1,50 | 1,50 |
| DC EL-8040^{®} | 0,90 | 0,90 | 0,90 |
| Glycerin | 5,00 | 5,00 | 5,00 |
| 1,6-Hexandiol | 6,00 | 6,00 | 6,00 |
| Tego Carbomer^{®} 140 | 0,40 | 0,40 | 0,40 |
| Betafin^{®} BP 20 | 2,00 | - | 2,00 |
| DSC C N^{®} | 2,00 | - | 2,00 |
| Phenonip^{®} ME | 0,50 | 0,50 | 0,50 |
| Allantoin | 0,05 | 0,05 | 0,05 |
| D-Panthenol 75% | 0,75 | 0,75 | 0,75 |
| alpha-Bisabolol | 0,05 | 0,05 | 0,05 |
| Aqua-Osmoline^{®} | - | 1,00 | 1,00 |
| Parfum | 0,30 | 0,30 | 0,30 |
| Natriumhydroxid | 0,05 | 0,05 | 0,05 |
| Simulgel^{®} EPG | 1,00 | 1,00 | 1,00 |
| Wasser | ad 100 | ad 100 | ad 100 |

Die nachfolgenden Tabellen 4 und 5 enthalten die Zusammensetzungen weiterer erfindungsgemäßer Mittel.

**Tabelle 4**

| **W/O-Emulsion; Rohstoffe** | **[Gew.-%]** |
|---|---|
| Dehymuls^{®} PGPH | 1,00 |
| Monomuls^{®} 90 O 18 | 1,00 |
| Zincum^{®} N29 Cosmetic | 1,50 |
| Lameform^{®} TGI | 2,00 |
| Softisan^{®} 649 | 1,00 |
| Paraffin wax, microcrystalline | 0,90 |
| Cetiol^{®} CC | 1,50 |
| Cetiol^{®} SN | 5,00 |
| Lanolin | 5,00 |
| Caprylic/Capric Triglyceride | 2,00 |
| Matrixyl^{®} 3000 Lipo | 3,00 |
| Vitamin E-acetate | 0,50 |
| Vitamin A-palmitate | 0,03 |
| Bienenwachs | 0,80 |
| Glycerin 86% | 5,00 |
| Betafin^{®} BP 20 | 2,00 |
| DSH-C N^{®} | 2,00 |
| Aqua-Osmoline^{®} | 1,00 |
| Magnesiumsulfat * 7H₂O | 1,00 |
| RonaCare^{®} AP | 0,40 |
| Crossential^{®} SA 14 | 0,50 |
| Amaranthsamenöl | 0,10 |
| Cosmedia^{®} Silc | 2,00 |
| NP Moist^{®} 24 | 3,00 |
| Konservierungsmittel, Parfum | q.s. |
| Wasser | ad 100 |

**Tabelle 5**

| **Wässriges Gel; Rohstoffe** | **[Gew.-%]** |
|---|---|
| Tego Carbomer^{®} 140 | 1,45 |
| D-Panthenol, 75% | 1,35 |
| Propylenglycol | 7,00 |
| Masquol^{®} NTA-Na₃, liquid | 0,03 |
| Alpha-Bisabolol | 1,00 |
| Betafin^{®} BP 20 | 2,00 |
| DSH-C N^{®} | 2,00 |
| Aqua-Osmoline^{®} | 1,00 |
| Pfefferminzextrakt | 1,00 |
| NaOH | 0,86 |
| Sorbitol 70% | 4,00 |
| Ethanol 96% | 5,00 |
| DC^{®} 1401 Fluid | 3,00 |
| Natriumsalicylat | 0,50 |
| Konservierungsmittel, Parfum, Farbstoff | q.s. |
| Wasser | ad 100 |

### In den Tabellen 3, 4 und 5 wurden die folgenden Handelsprodukte verwendet:

*Montanov*^{®} *202* (INCI-Bezeichnung: Arachidyl Alcohol, Behenyl Alcohol, Arachidyl Glucoside), Seppic; *Cetiol*^{®} CC (INCI-Bezeichnung: Dicaprylyl Carbonate), BASF; *Controx*^{®} *KS* C (INCI-Bezeichnung: Tocopherol, Hydrogenated Palm Glycerides Citrate), BASF; *Cetiol*^{®} *SB 45* (INCI-Bezeichnung: (INCI-Bezeichnung: Shea Butter), BASF; *DC EL-8040*^{®} (INCI-Bezeichnung: Isododecane, Dimethicone Crosspolymer); Dow Corning; *Tego Carbomer*^{®} *140* (INCI-Bezeichnung: Carbomer), Evonik; *Betafin*^{®} *BP 20* (INCI-Bezeichnung: Betaine), Finnfeeds Finland Oy; *DSC C N*^{®} (INCI-Bezeichnung: Dimethylsilanol Hyaluronate), Exsymol; *Phenonip*^{®} *ME* (INCI-Bezeichnung: Phenoxyethanol, Methylparaben, Ethylparaben) Clariant; *Aqua-Osmoline*^{®} (INCI-Bezeichnung: Aqua, Glycerin, Ceratonia Siliqua (Carob) Seed Extract), ISP Vincience; *Simulgel*^{®} *EPG* (INCI-Bezeichnung: Sodium Acrylates/Sodium Aryloyldimethyl Taurate Copolymer, Polyisobutene, Caprylyl/Capryl Glucoside), Seppic; *Dehymuls*^{®} *PGPH* (INCI-Bezeichnung: Polyglyceryl-2-dipolyhydroxystearate), BASF; *Monomuls*^{®} *90 O* 18 (INCI-Bezeichnung: Glyceryl Oleate), BASF; *Zincum*^{®} *N* 29 (INCI-Bezeichnung: Zinc Stearate), Bärlocher; *Lameform*^{®} *TGI* (INCI-Bezeichnung: Polyglyceryl-3-diisostearate), BASF; *Softisan*^{®} *TGI* (INCI-Bezeichnung: Bis-Diglyceryl Polyacyladipate-2), Sasol; *Cetiol*^{®} *SN* (INCI-Bezeichnung: Cetearyl Isononanoate), BASF; *Matrixyl*^{®} *3000* (INCI-Bezeichnung: C12-15 Alkyl Benzoate, Sorbitan Laurate, Tristearin, Acetylated Glycol Stearate, Palmitoyl Oligopeptide, Palmitoyl Tetrapeptide-7), Sederma; *RonaCare*^{®} *AP* (INCI-Bezeichnung: Bis-Ethylhexyl Hydroxydimethoxy Benzylmalonate), Merck; *Crossential*^{®} *SA* 14 (INCI-Bezeichnung: Echium Plantagineum Seed Oil), Croda; *Cosmedia*^{®} *Silc* (INCI-Bezeichnung: Silica), BASF; *NP*^{®} *Moist* 24 (INCI-Bezeichnung: Imperata Cylindrica Root Extract , Glycerin, Aqua (Water), Caprylyl Glycol, Carbomer, Acrylates/C10-30 Alkyl Acrylate Crosspolymer), Sederma; *Masquol*^{®} *NTA-Na₃ liquid* (INCI-Bezeichnung: Nitrilotriacetic acid trisodium salt), Protex; *DC*^{®} *1401* (INCI-Bezeichnung: Cyclomethicone, Dimethiconol), Dow Corning

## Patentansprüche

1. Kosmetische Zusammensetzung zur topischen Behandlung der Haut, enthaltend in einem geeigneten kosmetischen Träger - bezogen auf ihr Gesamtgewicht -
a) 0,01 bis 20 Gew.-% mindestens eines Esters der Hyaluronsäure,
b) 0,01 bis 20 Gew.-% mindestens einer Verbindung der nachfolgenden Formel (I) 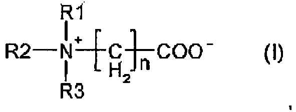 die ein Molekulargewicht von 75 bis 200 aufweist, und in der
n für eine Zahl von 1 bis 3 und
die Reste R1, R2 und R3 unabhängig voneinander für ein Wasserstoffatom, eine Methylgruppe, eine Ethylgruppe oder eine C₂-C₄-Hydroxyalkylgruppe stehen, und
c) 0,01 bis 20 Gew.-% mindestens eines wässrigen oder wassrig-alkoholischen Extrakts aus den Samen des Johannisbrotbaums *(Ceratonia siliqua).*

2. Kosmetische Zusammensetzung gemäß Anspruch 1, **dadurch gekennzeichnet, dass** sie den oder die Wirkstoff(e) a) in einer Gesamtmenge von 0,05 - 15 Gew.-%, bevorzugt 0,1 - 10 Gew.-%, besonders bevorzugt 0,5 - 7,5 Gew.-% und insbesondere 1 - 5 Gew.-%, jeweils bezogen auf das Gesamtgewicht der Zusammensetzung, enthält.

3. Kosmetische Zusammensetzung gemäß einem der Ansprüche 1 oder 2, **dadurch gekennzeichnet, dass** sie die Verbindung(en) der Formel (I) in einer Gesamtmenge von 0,05 - 15 Gew.-%, bevorzugt 0,1 - 10 Gew.-%, besonders bevorzugt 0,5 - 7,5 Gew.-% und insbesondere 1 - 5 Gew.-%, jeweils bezogen auf das Gesamtgewicht der Zusammensetzung, enthält

4. Kosmetische Zusammensetzung gemäß einem der Ansprüche 1 bis 3, **dadurch gekennzeichnet, dass** sie einen wässrigen oder einen wässrig-alkoholischen Extrakt aus den Samen des Johannisbrotbaums *(Ceratonia siliqua)* in einer Gesamtmenge von 0,05 - 15 Gew.-%, bevorzugt 0,1 - 10 Gew.-%, besonders bevorzugt 0,25 - 7,5 Gew.-% und insbesondere 0,5 - 5 Gew.-%, jeweils bezogen auf das Gesamtgewicht der Zusammensetzung, enthält.

5. Kosmetische Zusammensetzung gemäß Anspruch 4, **dadurch gekennzeichnet, dass** sie einen wässrig-alkoholischen Extrakt aus den Samen des Johannisbrotbaums *(Ceratonia siliqua)* enthält, der einen Proteingehalt im Bereich von 0,5 bis 10 g/l, bevorzugt von 1 bis 5 g/l und insbesondere von 1,5 bis 3,5 g/l in einer Mischung aus Wasser und einem Polyol, bevorzugt Glycerin, aufweist

6. Kosmetische Zusammensetzung gemäß einem der Ansprüche 1 bis 5, **dadurch gekennzeichnet, dass** sie als Wirkstoff a) mindestens einen Silanolester der Hyaluronsäure, bevorzugt Dimethylsilanediol hyaluronate (INCI-Bezeichnung: Dimethylsilanol Hyaluronate), enthält.

7. Kosmetische Zusammensetzung gemäß einem der Ansprüche 1 bis 6, dadurch gekenntzeichnet, dass sie mindestens eine Verbindung der Formel (I) enthält, in der die Reste R1, R2 und R3 jeweils für eine Methylgruppe stehen und n für die Zahl 1 steht (Trimethylglycin).

8. Kosmetische Zusammensetzung gemäß einem der vorstehenden Ansprüche, **dadurch gekennzeichnet, dass** sie in Form einer Öl-in-Wasser-Emulsion, einer Wasser-in-Öl-Emulsion und/oder in der Form eines wässrigen Gels vorliegt.

9. Nicht-therapeutische, kosmetische Verwendung einer kosmetischen topischen Zusammensetzung, die in einem geeigneten kosmetischen Träger - bezogen auf ihr Gesamtgewicht -
a) 0,01 bis 20 Gew.-% mindestens eines Esters der Hyaluronsäure,
b) 0,01 bis 20 Gew.-% mindestens einer Verbindung der nachfolgenden Formel (1) 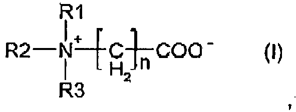 die ein Molekulargewicht von 75 bis 200 aufweist, und in der n für eine Zahl von 1 bis 3 und
die Reste R1, R2 und R3 unabhängig voneinander für ein Wasserstoffatom, eine Methylgruppe, eine Ethylgruppe oder eine C₂-C₄-Hydroxyalkylgruppe stehen, und
c) 0,01 bis 20 Gew.-% mindestens eines wässrigen oder wässrig-alkoholischen Extrakts aus den Samen des Johannisbrotbaums *(Ceratonia siliqua).*
zur Steigerung der mechanischen Festigkeit der Epidermis,
zur Verbesserung der Barriereeigenschaften der Epidermis,
zur nachhaltigen Steigerung der Hautfeuchte,
zur Verbesserung der Zell-Zell-Verbindungen in der Epidermis,
zum Schutz der Epidermis vor mechanischen Verletzungen und/oder vor exogenen Noxen, zur nicht-therapeutischen, kosmetischen Behandlung intrinsisch und/oder extrinsisch gealterter Haut, insbesondere zur Antifaltenbehandlung,
zur Glättung der Haut und/oder zur Verminderung von Falten, Fältchen und/oder feinen Linien und/oder zur Verbesserung des optischen Erscheinungsbildes der Haut.

10. Nicht-therapeutische, kosmetische Verwendung gemäß Anspruch 9, **dadurch gekennzeichnet, dass** die hauteigene Hyaluronsäureproduktion sowie die Produktion der Proteine Filaggrin, Aquaporin und Claudin-1 gesteigert wird.

## Claims

1. A cosmetic composition for topical treatment of the skin, containing, in a suitable cosmetic carrier, based on its total weight:
a) 0.01 to 20 wt.% of at least one hyaluronic acid ester,
b) 0.01 to 20 wt.% of at least one compound of the following formula (I) 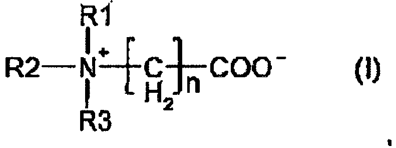 that has a molecular weight of between 75 and 200, and in which
n represents a number from 1 to 3, and
the functional groups R1, R2 and R3 represent, independently of one another, a hydrogen atom, a methyl group,
an ethyl group or a C₂-C₄ hydroxyalkyl group, and
c) 0.01 to 20 wt.% of at least one aqueous or aqueous-alcoholic extract from the seeds of the locust bean *(Ceratonia siliqua).*

2. The cosmetic composition according to claim 1, **characterized in that** it contains the active ingredient(s) a) in a total amount of from 0.05 to 15 wt.%, preferably from 0.1 to 10 wt.%, particularly preferably from 0.5 to 7.5 wt.%, and in particular from 1 to 5 wt.%, based in each case on the total weight of the composition.

3. The cosmetic composition according to either claim 1 or claim 2, **characterized in that** it contains the compound(s) of formula (I) in a total amount of from 0.05 to 15 wt.%, preferably from 0.1 to 10 wt.%, particularly preferably from 0.5 to 7.5 wt.%, and in particular from 1 to 5 wt.%, based in each case on the total weight of the composition.

4. The cosmetic composition according to one of claims 1 to 3, **characterized in that** it contains an aqueous or aqueous-alcoholic extract from the seeds of the locust bean *(Ceratonia siliqua)* in a total amount of from 0.05 to 15 wt.%, preferably from 0.1 to 10 wt.%, particularly preferably from 0.25 to 7.5 wt.%, and in particular from 0.5 to 5 wt.%, based in each case on the total weight of the composition.

5. The cosmetic composition according to claim 4, **characterized in that** it contains an aqueous-alcoholic extract from the seeds of the locust bean *(Ceratonia siliqua)* that has a protein content in the range of from 0.5 to 10 g/l, preferably from 1 to 5 g/l, and in particular from 1.5 to 3.5 g/l in a mixture of water and a polyol, preferably glycerol.

6. The cosmetic composition according to one of claims 1 to 5, **characterized in that** it contains at least one hyaluronic acid silanol ester, preferably dimethylsilanediol hyaluronate (INCI name: Dimethylsilanol Hyaluronate), as the active ingredient a).

7. The cosmetic composition according to one of claims 1 to 6, **characterized in that** it contains at least one compound of formula (I) in which the functional groups R1, R2 and R3 each represent a methyl group, and n represents the number 1 (trimethylglycine).

8. The cosmetic composition according to one of the preceding claims, **characterized in that** it is in the form of an oil-in-water emulsion, a water-in-oil emulsion and/or in the form of an aqueous gel.

9. The non-therapeutic, cosmetic use of a cosmetic topical composition containing, in a suitable cosmetic carrier, based on its total weight:
a) 0.01 to 20 wt.% of at least one hyaluronic acid ester,
b) 0.01 to 20 wt.% of at least one compound of the following formula (I) 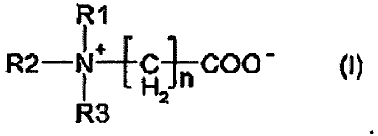 that has a molecular weight of between 75 and 200, and in which
n represents a number from 1 to 3, and
the functional groups R1, R2 and R3 represent, independently of one another, a hydrogen atom, a
methyl group, an ethyl group or a C₂-C₄ hydroxyalkyl group, and
c) 0.01 to 20 wt.% of at least one aqueous or aqueous-alcoholic extract from the seeds of the locust bean *(Ceratonia siliqua)*
for increasing the mechanical strength of the epidermis,
for improving the barrier properties of the epidermis,
for increasing the skin moisture in a lasting manner,
for improving the cell-cell connections in the epidermis,
for protecting the epidermis from mechanical damage and/or from exogenous noxae,
for non-therapeutic, cosmetic treatment of intrinsically and/or extrinsically aged skin, in particular for anti-wrinkle treatment,
for smoothing the skin and/or for reducing wrinkles, crow's feet and/or fine lines,
and/or for improving the optical appearance of the skin.

10. The non-therapeutic, cosmetic use according to claim 9, **characterized in that** the skin's own hyaluronic acid production and the production of the proteins filaggrin, aquaporin and claudin-1 is increased.

## Revendications

1. Composition cosmétique pour le traitement topique de la peau, contenant dans un vecteur cosmétique adéquat - rapporté à son poids total -
a. 0,01 à 20 % en poids d'au moins un ester de l'acide hyaluronique,
b. 0,01 à 20 % en poids d'au moins un composé selon la formule suivante (I) 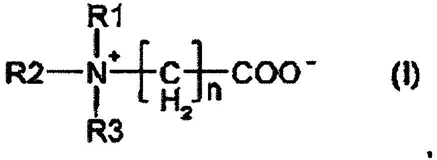 présentant un poids moléculaire de 75 à 200, et où
n représente un chiffre de 1 à 3,
les résidus R1, R2 et R3 représentent, indépendamment les uns des autres, un atome d'hydrogène, un groupe méthyle, un groupe éthyle ou un groupe hydroxyalkyle en C₂₋₄, et
c. 0,01 à 20 % en poids d'au moins un extrait aqueux ou hydro-alcoolique de graines de caroubier *(ceratonia siliqua).*

2. Composition cosmétique selon la revendication 1, **caractérisée en ce que** contient la ou les substances actives a) dans une quantité totale de 0,05 à 15 % en poids, préférentiellement de 0,1 à 10 % en poids, particulièrement préférentiellement de 0,5 à 7,5 % en poids et en particulier de 1 à 5 % en poids rapporté au poids total de la composition.

3. Composition cosmétique selon l'une des revendications 1 ou 2, **caractérisée en ce qu'**elle contient le ou les composés de formule (I) dans une quantité totale de 0,05 à 15 % en poids, préférentiellement de 0,1 à 10 % en poids, particulièrement préférentiellement de 0,5 à 7,5 % en poids et en particulier de 1 à 5 % en poids rapporté au poids total de la composition.

4. Composition cosmétique selon l'une des revendications 1 à 3, **caractérisée en ce qu'**elle contient un extrait aqueux ou hydro-alcoolique de graines de caroubier *(ceratonia siliqua)* dans une quantité totale de 0,05 à 15 % en poids, préférentiellement de 0,1 à 10 % en poids, particulièrement préférentiellement de 0,25 à 7,5 % en poids et en particulier de 0,5 à 5 % en poids rapporté au poids total de la composition.

5. Composition cosmétique selon la revendication 4, **caractérisée en ce qu'**elle contient un extrait aqueux ou hydro-alcoolique de graines de caroubier *(ceratonia siliqua)* présentant une teneur en protéines dans la gamme de 0,5 à 10 g/l, préférentiellement de 1 à 5 g/l et en particulier de 1,5 à 3,5 g/l dans un mélange d'eau et d'un polyol, préférentiellement de glycérine.

6. Composition cosmétique selon l'une des revendications 1 à 5, **caractérisée en ce qu'**elle contient comme agent actif a) au moins un silanoester de l'acide hyaluronique, préférentiellement le hyaluronate de diméthylsilyle (description INCI: dimethylsilanol hyaluronate).

7. Composition cosmétique selon l'une des revendications 1 à 5, **caractérisée en ce qu'**elle contient au moins un composé de formule (I), où les résidus R1, R2 et R3 représentent respectivement un groupe méthyle, et n = 1 (triméthylglycine).

8. Composition cosmétique selon l'une des revendications précédentes, **caractérisée en ce qu'**elle est présente sous forme d'une émulsion huile dans l'eau, d'une émulsion eau dans l'huile et/ou d'un gel aqueux.

9. Utilisation cosmétique non-thérapeutique d'une composition cosmétique topique, contenant dans un vecteur cosmétique adéquat - rapporté à son poids total -
a. 0,01 à 20 % en poids d'au moins un ester de l'acide hyaluronique,
b. 0,01 à 20 % en poids d'au moins un composé selon la formule suivante (I) 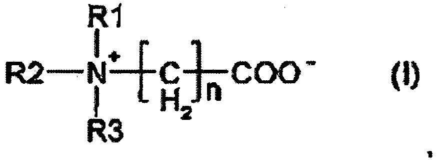 présentant un poids moléculaire de 75 à 200, et où
n représente un chiffre de 1 à 3,
les résidus R1, R2 et R3 représentent, indépendamment les uns des autres, un atome d'hydrogène, un groupe méthyle, un groupe éthyle ou un groupe hydroxyalkyle en C₂₋₄, et
c. 0,01 à 20 % en poids d'au moins un extrait aqueux ou hydro-alcoolique de graines de caroubier (*ceratonia siliqua*),
pour augmenter la résistance mécanique de l'épiderme,
pour améliorer les propriétés de barrière de l'épiderme,
pour augmenter durablement l'humidité de la peau,
pour améliorer les liaisons de cellule à cellule dans l'épiderme,
pour protéger l'épiderme contre les blessures mécaniques et/ou les substances toxiques exogènes,
pour le traitement cosmétique non-thérapeutique de la peau vieillie intrinsèquement et/ou extrinsèquement, en particulier pour le traitement contre les rides,
pour lisser la peau et/ou réduire les rides, ridules et/ou lignes fines et/ou pour améliorer l'aspect optique de la peau.

10. Utilisation cosmétique non-thérapeutique selon la revendication 9, **caractérisée en ce que** la production d'acide hyaluronique de la peau ainsi que la production des protéines filaggrine, aquaporine et claudine 1 sont augmentées.
